# EUROPEAN PATENT APPLICATION

(11) **EP 3 267 428 A1**
(43) Date of publication of application: **10.01.2018**
(21) Application number: 16759189.0
(22) Date of filing: 04.03.2016
(51) Int. Cl.: G09F 19/00, A61B 6/00, A61B 5/00

(54) **MEDICAL IMAGE SYSTEM AND METHOD FOR OPERATING MEDICAL IMAGE SYSTEM**

(30) Priority: 04.03.2015 KR 20150030553
(71) Applicant: Samsung Electronics Co., Ltd., Gyeonggi-do 16677 (KR)
(72) Inventor: HONG, Jin-beom, Seoul 02423 (KR); KIM, Seung-hoon, Suwon-si Gyeonggi-do 16690 (KR); KIM, Jung-woo, Suwon-si Gyeonggi-do 16707 (KR)
(74) Representative: Gulde & Partner
(86) International application number: PCT/KR2016/002216
(87) International publication number: WO 2016/140553

(57) **Abstract**

Provided is a monitoring device that comprises; a port connected to a network interconnecting a plurality of units of an X-ray apparatus, and configured to transmit or receive data to or from each of the plurality of units; and a controller configured to control the port to perform communication with each of the plurality of units, and to monitor operational states of the plurality of units, based on the data.

## Description

### TECHNICAL FIELD

The present disclosure relates to a medical imaging system and a method of operating the medical imaging system.

### BACKGROUND ART

In general, X-rays are electromagnetic waves having a wavelength of 0.01 to 100 Å and can pass through an object. Thus, they may be commonly used in a wide range of applications, such as medical equipment that take images of the inside of a living body and non-destructive testing equipment for industrial use.

X-ray imaging apparatuses using X-rays allow X-rays emitted by an X-ray source to pass through an object, and detect a difference between the intensities of the passed X-rays from an X-ray detector to thereby acquire an X-ray image of the object. X-ray imaging apparatuses also easily identify the internal structure of an object based on an X-ray image of the object and diagnose a disease of the object.

### DETAILED DESCRIPTION OF THE INVENTION

### TECHNICAL PROBLEM

Operational states of units of a medical image processing apparatus are monitored, and monitoring results are displayed.

A web server is loaded into the medical image processing apparatus so that a wide range of web services of the medical image processing apparatus is provided.

Technical problems of an embodiment of the present disclosure are not limited to the aforementioned technical problems, and other unstated technical problems may be inferred from embodiments below.

### TECHNICAL SOLUTION

According to an aspect of the present disclosure, there is provided a monitoring device that comprises; a port connected to a network interconnecting a plurality of units of an X-ray apparatus, and configured to transmit or receive data to or from each of the plurality of units; and a controller configured to control the port to perform communication with each of the plurality of units, and to monitor operational states of the plurality of units, based on the data.

### ADVANTAGEOUS EFFECTS

A monitoring device may be used to determine existence or non-existence of an operational error in an X-ray apparatus and to examine failure in the X-ray apparatus. The monitoring device may check an operational state of the X-ray apparatus, and may control a unit of the X-ray apparatus according to existence or non-existence of an error in the operational state. The monitoring device may not be connected to each of the plurality of units of the X-ray apparatus but may be connected to a position having an easy access and not having a driving unit. If the monitoring device is connected to a unit having a driving unit, the monitoring device has to move together whenever the unit having a driving unit moves. Thus, the monitoring device is connected to the position not having a driving unit, thereby rapidly and efficiently inspecting the X-ray apparatus.

### DESCRIPTION OF THE DRAWINGS

The present disclosure will now be described more fully through the detailed descriptions below with reference to the accompanying drawings, in which reference numerals denote structural elements.
FIG. 1 illustrates a configuration of an X-ray system related to the present disclosure, according to an embodiment.
FIG. 2 is a perspective view of a fixed-type X-ray apparatus related to the present disclosure, according to an embodiment.
FIG. 3 illustrates a mobile X-ray apparatus related to the present disclosure, according to an embodiment.
FIG. 4 illustrates a detailed configuration of a detector related to the present disclosure, according to an embodiment.
FIG. 5 is a conceptual diagram for describing a method of monitoring a plurality of units of an X-ray apparatus, according to an embodiment.
FIG. 6A is a block diagram illustrating a configuration of a monitoring device, according to an embodiment.
FIG. 6B is a block diagram illustrating a configuration of the monitoring device, according to another embodiment.
FIG. 7 is a diagram for describing a screen image displaying a monitoring result, according to an embodiment.
FIG. 8 is a diagram for describing a screen image displaying a monitoring result, according to another embodiment.
FIG. 9 is a diagram for describing a screen image for receiving a user input of controlling the X-ray apparatus, according to an embodiment.
FIG. 10 is a flowchart of an operating method performed by a monitoring device, according to an embodiment.
FIG. 11 is a flowchart of an operating method performed by the monitoring device, according to another embodiment.
FIG. 12 is a flowchart of an operating method performed by the monitoring device, according to another embodiment.
FIG. 13 is a block diagram illustrating a configuration of an X-ray apparatus, according to an embodiment.
FIG. 14A is a diagram for describing a web interface provided by a web server, according to an embodiment.
FIG. 14B is a diagram for describing a web interface provided by the web server, according to another embodiment.
FIG. 14C is a diagram for describing a web interface provided by the web server, according to another embodiment.
FIG. 15 is a diagram for describing types of a service provided by the web server, according to an embodiment.
FIG. 16 is a flowchart of an operating method performed by the X-ray apparatus, according to an embodiment.

### BEST MODE

According to an aspect of the present disclosure, there is provided a monitoring device comprising; a port connected to a network interconnecting a plurality of units of an X-ray apparatus, and configured to transmit or receive data to or from each of the plurality of units; and a controller configured to control the port to perform communication with each of the plurality of units, and to monitor operational states of the plurality of units, based on the data.

The controller is further configured to control the port to receive first data comprising an operational state of a first unit from among the plurality of units.

The controller is further configured to determine the operational state of the first unit, based on the first data, and to generate an operation command for the first unit according to the determined operational state.

The port is further configured to transmit the operation command for the first unit to the first unit.

The data uses at least one protocol from among a controller area network (CAN), a local interconnect network (LIN), a FlexRay, and a media oriented system transport (MOST).

The monitoring device further comprises a display device configured to display the monitoring result.

The display device is further configured to display a screen image displaying the plurality of units of the X-ray apparatus on a screen of the monitoring device, and to display the data on the screen image.

The plurality of units displayed on the screen image are arranged based on actual placement of the plurality of units.

The display device is further configured to display a screen image displaying a setting status or an operational state of at least one unit from among the plurality of units.

The display device comprises an input unit configured to receive a user input of controlling the setting status or the operational state, and the controller is further configured to control the setting status or the operational state of the at least one unit, based on the user input.

The port is connected to a link to a first unit from among the plurality of units.

The monitoring device further comprises a communication unit configured to transmit the data to an external apparatus via another network different from the network.

According to another aspect of the present disclosure, there is provided an X-ray apparatus comprising: an X-ray radiator configured to radiate an X-ray; a detector configured to detect the X-ray that has been radiated from the X-ray radiator and transmitted through an object; and a controller configured to control the X-ray radiator to adjust the radiated X-ray, and to control the detector to detect the X-ray, wherein a web server configured to provide at least one of setting information, operational state information, and control information, which are with respect to the X-ray apparatus, is installed in the controller.

The web server is further configured to provide different web interfaces according to groups of users using the X-ray apparatus.

The X-ray apparatus further comprises: a display configured to display the web interfaces; and an input unit configured to receive a user input via the web interfaces, wherein the controller is further configured to control the X-ray apparatus, based on the user input.

According to another aspect of the present disclosure, there is provided an operating method performed by a monitoring device, the operating method including receiving data from at least one unit from among a plurality of units of an X-ray apparatus via a network interconnecting the plurality of units of the X-ray apparatus; and monitoring an operational state of the at least one unit, based on the data.

The monitoring of the operational state of the at least one unit based on the data may include determining the operational state of the at least one unit based on the data; and generating an operation command for the at least one unit according to the determined operational state.

The operating method may further include transmitting the operation command for the at least one unit to the at least one unit.

The operating method may further include displaying a result of the monitoring.

The displaying of the result of the monitoring may include displaying the data on a screen image displaying the plurality of units of the X-ray apparatus.

The displaying of the result of the monitoring may include displaying a screen image displaying a setting status or an operational state of the at least one unit from among the plurality of units.

The operating method may further include receiving a user input of controlling the setting status or the operational state; and controlling the setting status or the operational state of the at least one unit, based on the user input.

According to another aspect of the present disclosure, there is provided an operating method performed by an X-ray apparatus in which a web server is installed, the operating method including displaying a web interface of the web server providing at least one of setting information, operational state information, and control information, which are with respect to the X-ray apparatus; receiving a user input via the web interface; and controlling the X-ray apparatus, based on the user input.

The displaying of the web interface may include providing different web interfaces according to groups of users using the X-ray apparatus.

### MODE OF THE INVENTION

Advantages and features of one or more embodiments of the present disclosure and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the present disclosure will only be defined by the appended claims.

Hereinafter, the terms used in the specification will be briefly described, and then the present disclosure will be described in detail.

The terms used in this specification are those general terms currently widely used in the art in consideration of functions regarding the inventive concept, but the terms may vary according to the intention of one of ordinary skill in the art, precedents, or new technology in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

Throughout the specification, an "image" may denote multi-dimensional data composed of discrete image elements (for example, pixels in a two-dimensional image and voxels in a three-dimensional image). For example, an image may be a medical image of an object acquired by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may include an organ (for example, the liver, the heart, the womb, the brain, breasts, or the abdomen), blood vessels, or a combination thereof. The object may be a phantom. The phantom denotes a material having a volume, a density, and an effective atomic number that are approximately the same as those of a living organism. For example, the phantom may be a spherical phantom having similar properties to those of the human body.

Throughout the specification, a "user" may be, but is not limited to, a medical expert, for example, a medical doctor, a nurse, a medical laboratory technologist, or a medical imaging expert, or a technician who repairs medical apparatuses.

An X-ray apparatus is a medical imaging apparatus that acquires images of internal structures of an object by transmitting an X-ray through the human body. The X-ray apparatus may acquire medical images of an object more simply within a shorter time than other medical imaging apparatuses including an MRI apparatus and a CT apparatus. Therefore, the X-ray apparatus is widely used in simple chest imaging, simple abdomen imaging, simple skeleton imaging, simple nasal sinuses imaging, simple neck soft tissue imaging, and breast imaging.

While such terms as "first," "second," etc., may be used to describe various components, such components must not be limited to the above terms. The above terms are used only to distinguish one component from another. For example, a first component discussed below could be termed a second component, and similarly, a second component may be termed a first component without departing from the teachings of this disclosure. The term "and/or" includes any and all combinations of one or more of the associated listed items.

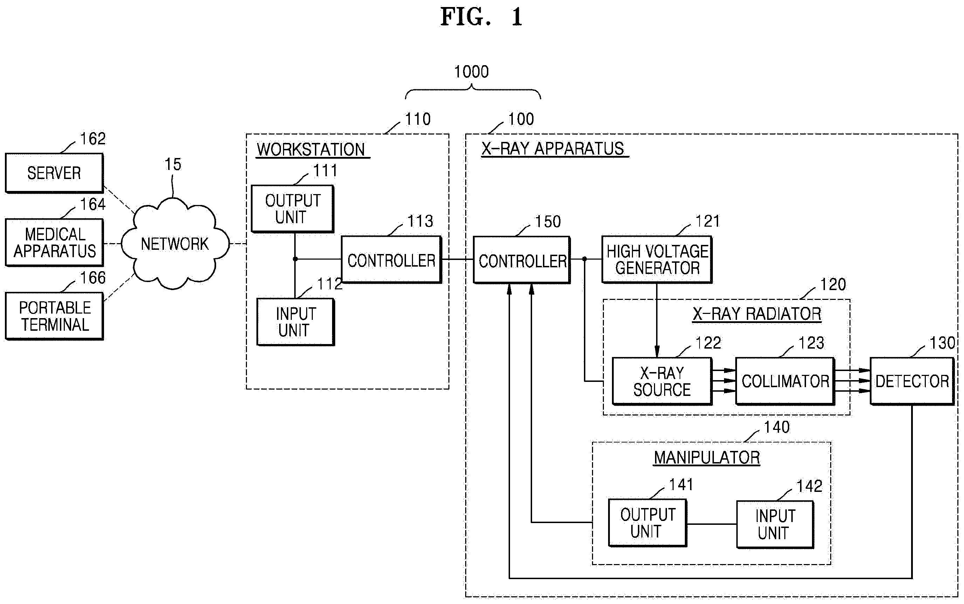

FIG. 1 is a block diagram of an X-ray system 1000.

Referring to FIG. 1, the X-ray system 1000 includes an X-ray apparatus 100 and a workstation 110. The X-ray apparatus 100 shown in FIG. 1 may be a fixed-type X-ray apparatus or a mobile X-ray apparatus. The X-ray apparatus 100 may include an X-ray radiator 120, a high voltage generator 121, a detector 130, a manipulator 140, and a controller 150. The controller 150 may control overall operations of the X-ray apparatus 100.

The high voltage generator 121 generates a high voltage for generating X-rays, and applies the high voltage to an X-ray source 122.

The X-ray radiator 120 includes the X-ray source 122 receiving the high voltage from the high voltage generator 121 to generate and radiate X-rays, and a collimator 123 for guiding a path of the X-ray radiated from the X-ray source 122 and adjusting an irradiation region radiated by the X-ray.

The X-ray source 122 includes an X-ray tube that may be realized as a vacuum tube diode including a cathode and an anode. An inside of the X-ray tube is set as a high vacuum state of about 10 mmHg, and a filament of the anode is heated to a high temperature to generate thermal electrons. The filament may be a tungsten filament, and a voltage of about 10V and a current of about 3 to 5 A may be applied to an electric wire connected to the filament to heat the filament.

In addition, when a high voltage of about 10 to about 300 kVp is applied between the cathode and the anode, the thermal electrons are accelerated to collide with a target material of the cathode, and then, an X-ray is generated. The X-ray is radiated outside via a window, and the window may be formed of a beryllium thin film. In this case, most of the energy of the electrons colliding with the target material is consumed as heat, and remaining energy is converted into the X-ray.

The cathode is mainly formed of copper, and the target material is disposed opposite to the anode. The target material may be a high resistive material such as chromium (Cr), iron (Fe), cobalt (Co), nickel (Ni), tungsten (W), or molybdenum (Mo). The target material may be rotated by a rotating field. When the target material is rotated, an electron impact area is increased, and a heat accumulation rate per unit area may be increased to be at least ten times greater than that of a case where the target material is fixed.

The voltage applied between the cathode and the anode of the X-ray tube is referred to as a tube voltage, and the tube voltage is applied from the high voltage generator 121 and a magnitude of the tube voltage may be expressed by a crest value (kVp). When the tube voltage increases, a velocity of the thermal electrons increases, and accordingly, an energy of the X-ray (energy of photon) that is generated when the thermal electrons collide with the target material is increased. The current flowing in the X-ray tube is referred to as a tube current that may be expressed as an average value (mA). When the tube current increases, the number of thermal electrons emitted from the filament is increased, and accordingly, the X-ray dose (the number of X-ray photons) generated when the thermal electrons collide with the target material is increased.

Therefore, the energy of the X-ray may be adjusted according to the tube voltage, and the intensity of the X-ray or the X-ray dose may be adjusted according to the tube current and the X-ray exposure time.

The detector 130 detects an X-ray that has been radiated from the X-ray radiator 120 and has been transmitted through an object. The detector 130 may be a digital detector. The detector 130 may be implemented by using a thin film transistor (TFT) or a charge coupled device (CCD). Although the detector 130 is included in the X-ray apparatus 100 in FIG. 1, the detector 130 may be an X-ray detector that is a separate device capable of being connected to or separated from the X-ray apparatus 100.

The X-ray apparatus 100 may further include a manipulator 140 for providing a user with an interface for manipulating the X-ray apparatus 100. The manipulator 140 may include an output unit 141 and an input unit 142. The input unit 142 may receive from a user a command for manipulating the X-ray apparatus 100 and various types of information related to X-ray imaging. The controller 150 may control or manipulate the X-ray apparatus 100 according to the information received by the input unit 142. The output unit 141 may output sound representing information related to an imaging operation such as the X-ray radiation under the control of the controller 150.

The workstation 110 and the X-ray apparatus 100 may be connected to each other by wire or wirelessly. When they are connected to each other wirelessly, a device (not shown) for synchronizing clock signals with each other may be further included. The workstation 110 and the X-ray apparatus 100 may exist within physically separate spaces.

The workstation 110 may include an output unit 111, an input unit 112, and a controller 113. The output unit 111 and the input unit 112 provide a user with an interface for manipulating the workstation 110 and the X-ray apparatus 200. The controller 113 may control the workstation 110 and the X-ray apparatus 200.

The X-ray apparatus 100 may be controlled via the workstation 110 or may be controlled by the controller 150 included in the X-ray apparatus 100. Accordingly, a user may control the X-ray apparatus 100 via the workstation 110 or may control the X-ray apparatus 100 via the manipulator 140 and the controller 150 included in the X-ray apparatus 100. In other words, a user may remotely control the X-ray apparatus 100 via the workstation 110 or may directly control the X-ray apparatus 100.

Although the controller 113 of the workstation 110 is separate from the controller 150 of the X-ray apparatus 100 in FIG. 1, FIG. 1 is only an example. As another example, the controllers 113 and 150 may be integrated into a single controller, and the single controller may be included in only one of the workstation 110 and the X-ray apparatus 100. Hereinafter, the controllers 113 and 150 may denote the controller 113 of the workstation 110 and/or the controller 150 of the X-ray apparatus 100.

The output unit 111 and the input unit 112 of the workstation 110 may provide a user with an interface for manipulating the X-ray apparatus 100, and the output unit 141 and the input unit 142 of the X-ray apparatus 100 may also provide a user with an interface for manipulating the X-ray apparatus 100. Although the workstation 110 and the X-ray radiation apparatus 100 include the output units 111 and 141, respectively, and the input units 112 and 142, respectively, in FIG. 1, embodiments are not limited thereto. Only one of the workstation 110 and the X-ray apparatus 100 may include an output unit or an input unit.

Hereinafter, the input units 112 and 142 may denote the input unit 112 of the workstation 110 and/or the input unit 142 of the X-ray apparatus 100, and the output units 111 and 141 may denote the output unit 111 of the workstation 110 and/or the output unit 141 of the X-ray apparatus 100.

Examples of the input units 112 and 142 may include a keyboard, a mouse, a touch screen, a voice recognizer, a fingerprint recognizer, an iris recognizer, and other input devices which are well known to one of ordinary skill in the art. The user may input a command for radiating the X-ray via the input units 112 and 142, and the input units 112 and 142 may include a switch for inputting the command. The switch may be configured so that a radiation command for radiating the X-ray may be input only when the switch is pushed in two steps.

In other words, when the user pushes the switch, a prepare command for performing a pre-heating operation for X-ray radiation may be input, and in this state, when the user pushes the switch deeper, a radiation command for performing substantial X-ray radiation may be input. When the user manipulates the switch as described above, the controllers 113 and 150 generate signals corresponding to the commands input through the switch manipulation, that is, a prepare signal, and transmit the generated signals to the high voltage generator 121 generating a high voltage for generating the X-ray.

When the high voltage generator 121 receives the prepare signal from the controllers 113 and 150, the high voltage generator 121 starts a pre-heating operation, and when the pre-heating is finished, the high voltage generator 121 outputs a ready signal to the controllers 113 and 150. In addition, the detector 130 also needs to prepare to detect the X-ray, and thus the high voltage generator 121 performs the pre-heating operation and the controllers 113 and 150 transmit a prepare signal to the detector 130 so that the detector 130 may prepare to detect the X-ray transmitted through the object. The detector 130 prepares to detect the X-ray in response to the prepare signal, and when the preparing for the detection is finished, the detector 130 outputs a ready signal to the controllers 113 and 150.

When the pre-heating operation of the high voltage generator 121 is finished and the detector 130 is ready to detect the X-ray, the controllers 113 and 150 transmit a radiation signal to the high voltage generator 121, the high voltage generator 121 generates and applies the high voltage to the X-ray source 122, and the X-ray source 122 radiates the X-ray.

When the controllers 113 and 150 transmit the radiation signal to the high voltage generator 121, the controllers 113 and 150 may transmit a sound output signal to the output units 111 and 141 so that the output units 111 and 141 output a predetermined sound and the object may recognize the radiation of the X-ray. The output units 111 and 141 may also output a sound representing information related to photographing in addition to the X-ray radiation. In FIG. 1, the output unit 141 is included in the manipulator 140; however, the embodiments are not limited thereto, and the output unit 141 or a portion of the output unit 141 may be located elsewhere. For example, the output unit 141 may be located on a wall of an examination room in which the X-ray photographing of the object is performed.

The controllers 113 and 150 control locations of the X-ray radiator 120 and the detector 130, photographing timing, and photographing conditions, according to photographing conditions set by the user.

In more detail, the controllers 113 and 150 control the high voltage generator 121 and the detector 130 according to the command input via the input units 112 and 142 so as to control radiation timing of the X-ray, an intensity of the X-ray, and a region radiated by the X-ray. In addition, the control units 113 and 150 adjust the location of the detector 130 according to a predetermined photographing condition, and controls operation timing of the detector 130.

Furthermore, the controllers 113 and 150 generate a medical image of the object by using image data received via the detector 130. In detail, the controllers 113 and 150 may receive the image data from the detector 130, and then, generate the medical image of the object by removing noise from the image data and adjusting a dynamic range and interleaving of the image data.

The output units 111 and 141 may output the medical image generated by the controllers 113 and 150. The output units 111 and 141 may output information that is necessary for the user to manipulate the X-ray apparatus 100, for example, a user interface (UI), user information, or object information. Examples of the output units 111 and 141 may include a speaker, a printer, a cathode ray tube (CRT) display, a liquid crystal display (LCD), a plasma display panel (PDP), an organic light emitting diode (OLED) display, a field emission display (FED), a light emitting diode (LED) display, a vacuum fluorescent display (VFD), a digital light processing (DLP) display, a flat panel display (FPD), a three-dimensional 3D) display, a transparent display, and other various output devices well known to one of ordinary skill in the art.

The maximum depth shown in FIG. 1 may further include a communicator (not shown) that may be connected to a server 162, a medical apparatus 164, and a portable terminal 166 via a network 15.

The communicator may be connected to the network 15 by wire or wirelessly to communicate with the server 162, the medical apparatus 164, or the portable terminal 166. The communicator may transmit or receive data related to diagnosis of the object via the network 15, and may also transmit or receive medical images captured by the medical apparatus 164, for example, a CT apparatus, an MRI apparatus, or an X-ray apparatus. Moreover, the communicator may receive a medical history or treatment schedule of an object (e.g., a patient) from the server 162 to diagnose a disease of the object. Also, the communicator may perform data communication with the portable terminal 166 such as a mobile phone, a personal digital assistant (PDA), or a laptop computer of a medical doctor or a client, as well as the server 162 or the medical apparatus 164 in a hospital.

The communicator may include one or more elements enabling communication with external apparatuses. For example, the communicator may include a local area communication module, a wired communication module, and a wireless communication module.

The local area communication module refers to a module for performing local area communication with an apparatus located within a predetermined distance. Examples of local area communication technology may include, but are not limited to, a wireless local area network (LAN), Wi-Fi, Bluetooth, ZigBee, Wi-Fi Direct (WFD), ultra wideband (UWD), infrared data association (IrDA), Bluetooth low energy (BLE), and near field communication (NFC).

The wired communication module refers to a module for communicating by using an electric signal or an optical signal. Examples of wired communication technology may include wired communication techniques using a pair cable, a coaxial cable, and an optical fiber cable, and other wired communication techniques that are well known to one of ordinary skill in the art.

The wireless communication module transmits and receives a wireless signal to and from at least one selected from a base station, an external apparatus, and a server in a mobile communication network. Here, examples of the wireless signal may include a voice call signal, a video call signal, and various types of data according to text/multimedia messages transmission.

The X-ray apparatus 100 shown in FIG. 1 may include a plurality of digital signal processors (DSPs), an ultra-small calculator, and a processing circuit for special purposes (for example, high speed analog/digital (A/D) conversion, high speed Fourier transformation, and an array process).

In addition, communication between the workstation 110 and the X-ray apparatus 100 may be performed using a high speed digital interface, such as low voltage differential signalling (LVDS), asynchronous serial communication, such as a universal asynchronous receiver transmitter (UART), a low latency network protocol, such as error synchronous serial communication or a controller area network (CAN), or any of other various communication methods that are well known to one of ordinary skill in the art.

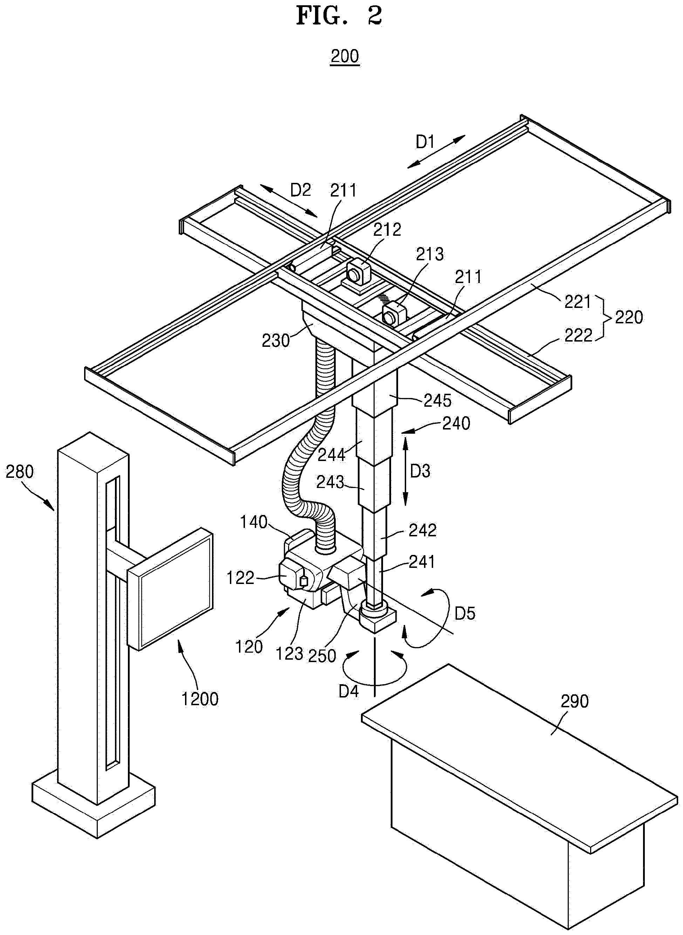

FIG. 2 is a perspective view of a fixed type X-ray apparatus 200. The fixed type X-ray apparatus 200 may be another embodiment of the X-ray apparatus 100 of FIG. 1. Components included in the fixed type X-ray apparatus 200 that are the same as those of the X-ray apparatus 100 of FIG. 1 use the same reference numerals, and repeated descriptions thereof will be omitted.

Referring to FIG. 2, the fixed type X-ray apparatus 200 includes a manipulator 140 providing a user with an interface for manipulating the X-ray apparatus 200, an X-ray radiator 120 radiating an X-ray to an object, a detector 130 detecting an X-ray that has passed through the object, first, second, and third motors 211, 212, and 213 providing a driving power to transport the X-ray radiator 120, a guide rail 220, a moving carriage 230, and a post frame 240. The guide rail 220, the moving carriage 230, and the post frame 240 are formed to transport the X-ray radiator 120 by using the driving power of the first, second, and third motors 211, 212, and 213.

The guide rail 220 includes a first guide rail 221 and a second guide rail 222 that are provided to form a predetermined angle with respect to each other. The first guide rail 221 and the second guide rail 222 may respectively extend in directions crossing each other at 90°.

The first guide rail 221 is provided on the ceiling of an examination room in which the X-ray apparatus 200 is disposed.

The second guide rail 222 is located under the first guide rail 221, and is mounted so as to slide along the first guide rail 221. A roller (not shown) that may move along the first guide rail 221 may be provided on the first guide rail 221. The second guide rail 222 is connected to the roller to move along the first guide rail 221.

A first direction D1 is defined as a direction in which the first guide rail 221 extends, and a second direction D2 is defined as a direction in which the second guide rail 222 extends. Therefore, the first direction D1 and the second direction D2 cross each other at 90°, and may be parallel to the ceiling of the examination room.

The moving carriage 230 is disposed under the second guide rail 222 so as to move along the second guide rail 222. A roller (not shown) moving along the second guide rail 222 may be provided on the moving carriage 230.

Therefore, the moving carriage 230 may move in the first direction D1 together with the second guide rail 222, and may move in the second direction D2 along the second guide rail 222.

The post frame 240 is fixed on the moving carriage 230 and located under the moving carriage 230. The post frame 240 may include a plurality of posts 241, 242, 243, 244, and 245.

The plurality of posts 241, 242, 243, 244, and 245 are connected to each other to be foldable, and thus, the post frame 240 may have a length that is adjustable in a vertical direction of the examination room while in a state of being fixed to the moving carriage 230.

A third direction D3 is defined as a direction in which the length of the post frame 240 increases or decreases. Therefore, the third direction D3 may be perpendicular to the first direction D1 and the second direction D2.

The detector 130 detects the X-ray that has passed through the object, and may be combined with a table type receptor 290 or a stand type receptor 280.

A rotating joint 250 is disposed between the X-ray radiator 120 and the post frame 240. The rotating joint 250 allows the X-ray radiator 120 to be coupled to the post frame 240, and supports a load applied to the X-ray radiator 120.

The X-ray radiator 120 connected to the rotating joint 250 may rotate on a plane that is perpendicular to the third direction D3. In this case, a rotating direction of the X-ray radiator 120 may be defined as a fourth direction D4.

Also, the X-ray radiator 120 may be configured to be rotatable on a plane perpendicular to the ceiling of the examination room. Therefore, the X-ray radiator 120 may rotate in a fifth direction D5 that is a rotating direction about an axis that is parallel with the first direction D1 or the second direction D2, with respect to the rotating joint 250.

The first, second, and third motors 211, 212, and 213 may be provided to move the X-ray radiator 120 in the first, second, and third directions D1, D2, and D3. The first, second, and third motors 211, 212, and 213 may be electrically driven, and the first, second, and third motors 211, 212, and 213 may respectively include an encoder.

The first, second, and third motors 211, 212, and 213 may be disposed at various locations in consideration of design convenience. For example, the first motor 211, moving the second guide rail 222 in the first direction D1, may be disposed around the first guide rail 221, the second motor 212, moving the moving carriage 230 in the second direction D2, may be disposed around the second guide rail 222, and the third motor 213, increasing or reducing the length of the post frame 240 in the third direction D3, may be disposed in the moving carriage 230. In another example, the first, second, and third motors 211, 212, and 213 may be connected to a driving power transfer unit (not shown) so as to linearly move the X-ray radiator 120 in the first, second, and third directions D1, D2, and D3. The driving power transfer unit may be a combination of a belt and a pulley, a combination of a chain and a sprocket, or a shaft, which are generally used.

In another example, motors (not shown) may be disposed between the rotating joint 250 and the post frame 240 and between the rotating joint 250 and the X-ray radiator 120 in order to rotate the X-ray radiator 120 in the fourth and fifth directions D4 and D5.

The manipulator 140 may be disposed on a side surface of the X-ray radiator 120.

FIG. 2 shows the fixed type X-ray apparatus 200 connected to the ceiling of the examination room, the fixed type X-ray apparatus 200 is merely an example for convenience of comprehension. That is, X-ray apparatuses according to embodiments of the present disclosure may include X-ray apparatuses having various structures that are well known to one of ordinary skill in the art, for example, a C-arm-type X-ray apparatus and an angiography X-ray apparatus, in addition to the fixed type X-ray apparatus 200 of FIG. 2.

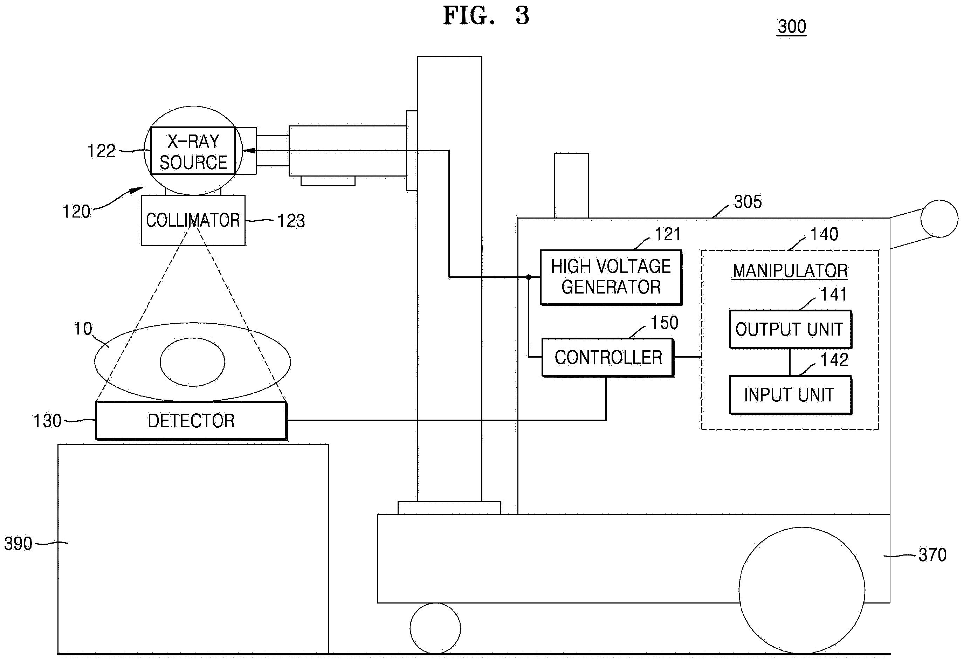

FIG. 3 is a diagram showing a configuration of a mobile X-ray apparatus 300 capable of performing an X-ray photographing operation regardless of a place where the photographing operation is performed. The mobile X-ray apparatus 300 may be another embodiment of the X-ray apparatus 100 of FIG. 1. Components included in the mobile X-ray apparatus 300 that are the same as those of the X-ray apparatus 100 of FIG. 1 use the same reference numerals as those used in FIG. 1, and a repeated description thereof will be omitted.

Referring to FIG. 3, the mobile X-ray apparatus 300 includes a transport unit 370 including a wheel for transporting the mobile X-ray apparatus 300, a main unit 305, an X-ray radiator 120, and a detector 130 detecting an X-ray that has been radiated from the X-ray radiator 120 toward an object and transmitted through the object. The main unit 305 includes a manipulator 140 providing a user with an interface for manipulating the mobile X-ray apparatus 300, a high voltage generator 121 generating a high voltage applied to an X-ray source 122, and a controller 150 controlling overall operations of the mobile X-ray apparatus 300. The X-ray radiator 120 includes the X-ray source 122 generating the X-ray, and a collimator 123 guiding a path along which the generated X-ray is emitted from the X-ray source 122 and adjusting an irradiation region radiated by the X-ray.

The detector 130 in FIG. 3 may not be combined with any receptor, and the detector 130 may be a portable detector which can exist anywhere.

In FIG. 3, the manipulator 140 is included in the main unit 305; however, embodiments are not limited thereto. For example, as illustrated in FIG. 2, the manipulator 140 of the mobile X-ray apparatus 300 may be disposed on a side surface of the X-ray radiator 120.

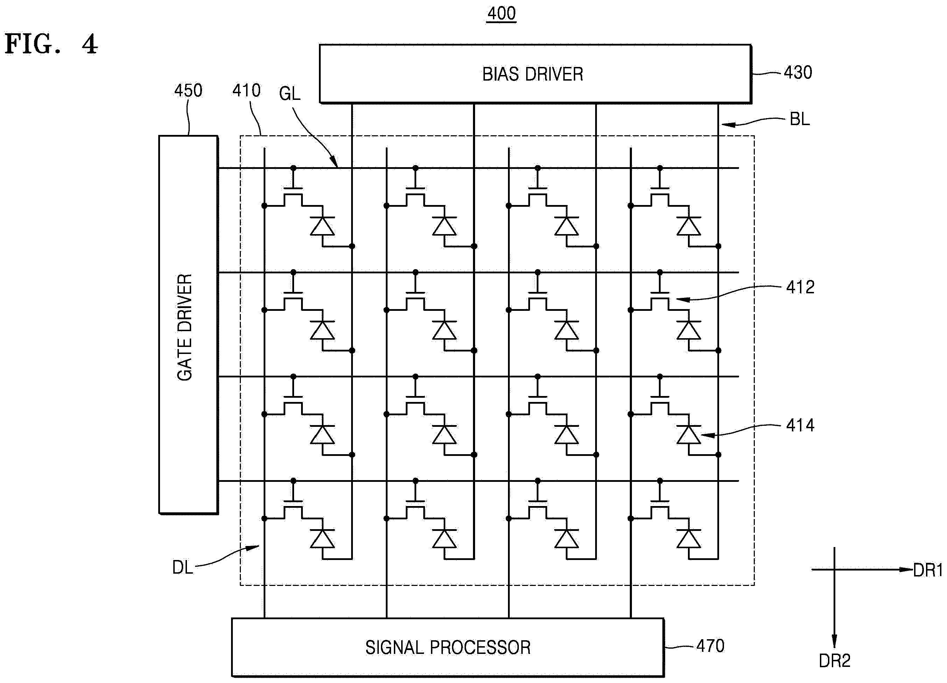

FIG. 4 is a block diagram illustrating a structure of the CT system 400. The detector 400 may be an embodiment of the detector 130 of FIGS. 1-3. The detector 400 may be an indirect type detector.

Referring to FIG. 4, the detector 400 may include a scintillator (not shown), a photodetecting substrate 410, a bias driver 430, a gate driver 450, and a signal processor 470.

The scintillator receives the X-ray radiated from the X-ray source 122 and converts the X-ray into light.

The photodetecting substrate 410 receives the light from the scitillator and converts the light into an electrical signal. The photodetecting substrate 410 may include gate lines GL, data lines DL, TFTs 412, photodiodes 414, and bias lines BL.

The gate lines GL may be formed in the first direction DR1, and the data lines DL may be formed in the second direction DR2 that crosses the first direction DR1. The first direction DR1 and the second direction DR2 may intersect perpendicularly to each other. FIG. 4 shows four gate lines GL and four data lines DL as an example.

The TFTs 412 may be arranged as a matrix in the first and second directions DR1 and DR2. Each of the TFTs 412 may be electrically connected to one of the gate lines GL and one of the data lines DL. A gate of the TFT 412 may be electrically connected to the gate line GL, and a source of the TFT 412 may be electrically connected to the data line DL. In FIG. 4, sixteen TFTs 412 (in a 4 x 4 arrangement) are shown as an example.

The photodiodes 414 may be arranged as a matrix in the first and second directions DR1 and DR2 so as to respectively correspond to the TFTs 412. Each of the photodiodes 414 may be electrically connected to one of the TFTs 412. An N-side electrode of each of the photodiodes 414 may be electrically connected to a drain of the TFT 412. FIG. 4 shows sixteen photodiodes 414 (in a 4 x 4 arrangement) as an example.

The bias lines BL are electrically connected to the photodiodes 414. Each of the bias lines BL may be electrically connected to P-side electrodes of an array of photodiodes 414. For example, the bias lines BL may be formed to be substantially parallel with the second direction DR2 so as to be electrically connected to the photodiodes 414. On the other hand, the bias lines BL may be formed to be substantially parallel with the first direction DR1 so as to be electrically connected to the photodiodes 414. FIG. 4 shows four bias lines BL formed along the second direction DR2 as an example.

The bias driver 430 is electrically connected to the bias lines BL so as to apply a driving voltage to the bias lines BL. The bias driver 430 may selectively apply a reverse bias voltage or a forward bias voltage to the photodiodes 414. A reference voltage may be applied to the N-side electrodes of the photodiodes 414. The reference voltage may be applied via the signal processor 470. The bias driver 430 may apply a voltage that is less than the reference voltage to the P-side electrodes of the photodiodes 414 so as to apply a reverse bias voltage to the photodiodes 414. On the other hand, the bias driver 430 may apply a voltage that is greater than the reference voltage to the P-side electrodes of the photodiodes 414 so as to apply a forward bias voltage to the photodiodes 414.

The gate driver 450 is electrically connected to the gate lines GL and thus may apply gate signals to the gate lines GL. For example, when the gate signals are applied to the gate lines GL, the TFTs 412 may be turned on by the gate signals. On the other hand, when the gate signals are not applied to the gate lines GL, the TFTs 412 may be turned off.

The signal processor 470 is electrically connected to the data lines DL. When the light received by the photodetecting substrate 410 is converted into the electrical signal, the electrical signal may be read out by the signal processor 470 via the data lines DL.

An operation of the detector 400 will now be described. During the operation of the detector 400, the bias driver 430 may apply the reverse bias voltage to the photodiodes 414.

While the TFTs 412 are turned off, each of the photodiodes 414 may receive the light from the scintillator and generate electron-hole pairs to accumulate electric charges. The amount of electric charge accumulated in each of the photodiodes 414 may correspond to the intensity of the received X-ray.

Then, the gate driver 450 may sequentially apply the gate signals to the gate lines GL along the second direction DR2. When a gate signal is applied to a gate line GL and thus TFTs 412 connected to the gate line GL are turned on, photocurrents may flow into the signal processor 470 via the data lines DL due to the electric charges accumulated in the photodiodes 414 connected to the turned-on TFTs 412.

The signal processor 470 may convert the received photocurrents into image data. The signal processor 470 may output the image data to the outside. The image data may be in the form of an analog signal or a digital signal corresponding to the photocurrents.

Although not shown in FIG. 4, if the detector 400 shown in FIG. 4 is a wireless detector, the detector 400 may further include a battery unit and a wireless communication interface unit.

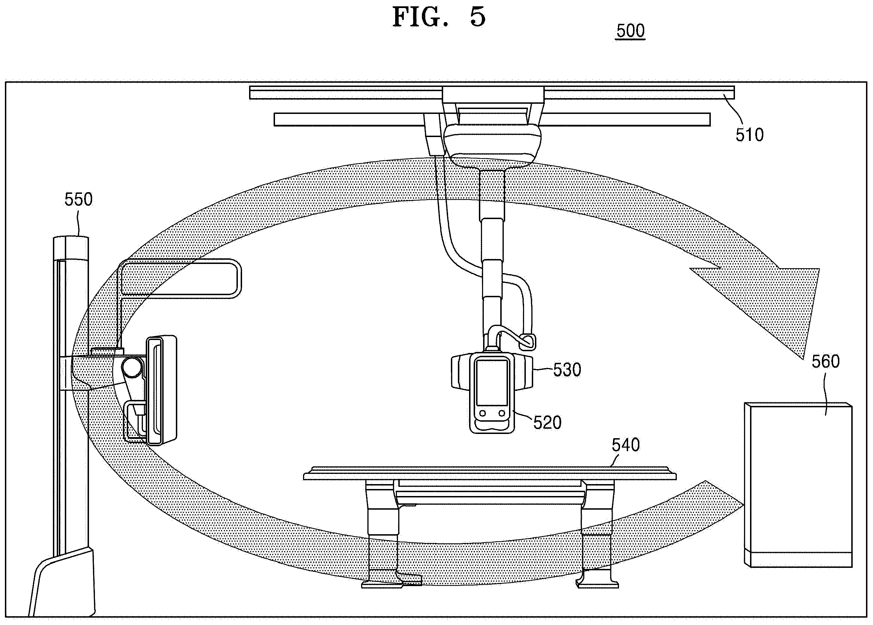

FIG. 5 is a conceptual diagram for describing a method of monitoring a plurality of units of an X-ray apparatus, according to an embodiment.

As illustrated in FIG. 5, the X-ray apparatus is an apparatus including a ceiling 510, a tube head unit (THU) 520, a collimator 530, a table 540, a stand 550, and a high voltage generator (HVG) power control unit (HPCU) 560, in which several units independently and organically operate to support a diagnosis based on image-capturing. In general, the X-ray apparatus operates in a manner that respective controllers for the ceiling 510, the THU 520, the collimator 530, the table 540, the stand 550, and the HPCU 560 perform control calculations at different locations, and exchange information.

A monitoring device may be used to determine existence or non-existence of an operational error in the X-ray apparatus and to examine failure in the X-ray apparatus. The monitoring device may check an operational state of the X-ray apparatus, and may control a unit of the X-ray apparatus according to existence or non-existence of an error in the operational state. The monitoring device may not be connected to each of the plurality of units of the X-ray apparatus but may be connected to a position having an easy access and not having a driving unit. If the monitoring device is connected to a unit having a driving unit, the monitoring device has to move together whenever the unit having a driving unit moves. Thus, the monitoring device is connected to the position not having a driving unit, thereby rapidly and efficiently inspecting the X-ray apparatus.

The monitoring device may be connected to the X-ray apparatus via a residual communication port that is not used in the X-ray apparatus or an extension communication adaptor. For particular example, the HPCU 560 may be located outside a shield room in which the X-ray apparatus is arranged, or may be located outside an operational range of a system. The monitoring device is connected to the HPCU 560 via a port, thereby monitoring separate operations of the units of the X-ray apparatus, and a mutual operation between the units.

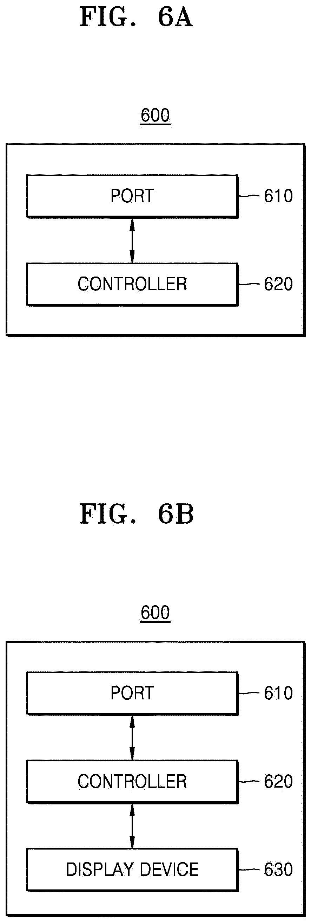

FIG. 6A is a block diagram illustrating a configuration of a monitoring device, according to an embodiment.

The monitoring device illustrated in FIG. 6A may be the X-ray apparatus 100, the workstation 110, the medical apparatus 164, the portable terminal 166, a medical imaging apparatus, a medical server, or any computing device capable of using and processing a medical image. In more detail, a controller of a monitoring device 600 illustrated in FIG. 6A may equally correspond to the controller 150 of the X-ray apparatus 100 or the controller 113 of the workstation 110 which is illustrated in FIG. 1. Here, descriptions which are the same as in FIG. 1 are omitted.

According to an embodiment, the monitoring device 600 may include a port 610 and a controller 620. It is obvious to one of ordinary skill in the art that common elements in addition to the elements illustrated in FIG. 6A may be further included.

The monitoring device 600 may monitor operational states of the plurality of units of the X-ray apparatus. A port 610 is connected to a network that interconnects the plurality of units. In addition, the port 610 exchanges data with each of the plurality of units. The port 610 may be connected to a link to a first unit from among the plurality of units. In more detail, the port 610 may be connected to an HPCU. The port 610 may receive, from each of the plurality of units, data including an operation state of the X-ray apparatus and a shared error.

The data may use at least one protocol from among a controller area network (CAN), a local interconnect network (LIN), a FlexRay, and a media oriented system transport (MOST).

The CAN refers to a network system developed to provide digital serial communication between various electronic control units (ECUs), which is cost-effective, is a very flexible network supporting a master/slave, multiple masters, a peer-to-peer, or the like, and provides high reliability to high temperature, shock, and a noisy environment.

The LIN is generally used in connecting intelligent actuators or sensors and does not require a high data transmission speed or complicated failure management, thereby supporting a data transmission speed of max. 19.6kBit/s. In addition, the LIN is based on a SCI(Scalable Coherent Interface) 8-bit interface, supports a single master/multiple slaves concept, and is configured of one master node and several (up to 15) slave nodes.

The FlexRay may be used in a steer-by-wire system, an x-by-wire system such as brake-by-wire, etc. which requires a high data transmission speed and excellent failure management. The FlexRay transmits data with maximal bandwidth of 10Mbit/s so that the FlexRay may be used in a real-time operation.

In addition, the FlexRay does not require a particular physical layer, thereby supporting all of copper line transmission media and optical fiber transmission media. Furthermore, the FlexRay supports a plurality of network topologies including a bus, a star, a cascaded star, a hybrid network topology, or the like.

A maximum transmission speed of a MOST bus is 24.8Mbit/s in a synchronous transmission mode and 14.4Mbit/s in an asynchronous transmission mode. The MOST bus has an additional asynchronous control channel that provides a data speed of max. 700kBit/s. A high data transmission speed of the MOST bus is appropriate for transmitting audio and video in real-time. In order to safely transmit data, the MOST bus uses, as a physical layer, an optical medium (a plastic optical fiber (POF)) which is not sensitive to electromagnetic compatibility (EMC).

The controller 620 controls the port 610 to perform communication with each of the plurality of units. The controller 620 monitors operational states of the plurality of units, based on data.

In more detail, the controller 620 controls the port 610 to receive first data including an operational state of the first unit from among the plurality of units. The controller 620 determines the operational state of the first unit, based on the first data, and generates an operation command for the first unit according to the determined operational state. The first unit receives the operation command, thereby performing a process corresponding to the operation command.

FIG. 6B is a block diagram illustrating a configuration of the monitoring device 600, according to another embodiment.

Compared to the monitoring device 600 illustrated in FIG. 6A, the monitoring device 600 illustrated in FIG. 6B may further include a display device 630.

In FIG. 6B, the display device 630 of the monitoring device 600 may equally correspond to the output unit 141 of the X-ray apparatus 100 or the output unit 111 of the workstation 110 which are illustrated in FIG. 1. In this regard, descriptions which are the same as in FIG. 1 are omitted.

The display device 630 displays a monitoring result. The display device 630 displays a screen image displaying the plurality of units of the X-ray apparatus. The display device 630 may display data on a screen. In this regard, the plurality of units displayed on the screen image are arranged based on actual placement of the plurality of units.

In addition, the display device 630 displays a screen image displaying a setting status or an operational state of at least one unit from among the plurality of units. The display device 630 may include an input unit to receive a user input of controlling the setting status or the operational state of the at least one unit. In this regard, the input unit may equally correspond to the input unit 142 of the X-ray apparatus 100 or the input unit 112 of the workstation 110 which are illustrated in FIG. 1. In this regard, descriptions which are the same as in FIG. 1 are omitted.

The input unit indicates a device to receive an input of data for controlling at least one unit from among the plurality of units of the X-ray apparatus. The input unit may include, but is not limited to, a hardware configuration such as a keypad, a mouse, a touch panel, a touch screen, a trackball, a jog switch, or the like. In addition, the input unit may further include various input means such as a voice recognition sensor, a gesture recognition sensor, a fingerprint recognition sensor, an iris recognition sensor, a depth sensor, a distance sensor, or the like.

The input unit receives a user input of changing the setting status or the operational state of the at least one unit from among the plurality of units of the X-ray apparatus. A controller controls the setting status or the operational state of the at least one unit, based on the user input.

In this regard, the setting status may refer to a status of a unit of the X-ray apparatus which is set to operate. Here, data indicating the setting status may include a parameter used in operating the unit and a default value of the parameter, and a parameter used in interoperation between the unit and another unit and a current value of the parameter.

In addition, the operational state may indicate whether at least one unit from among the plurality of units normally operates while the X-ray apparatus operates. Data indicating the operational state may include parameters related to units, respectively, and current values of the parameters, and it is obvious to one of ordinary skill in the art that the data may include another data.

The input unit may generate and output a user interface screen for receiving an input of a predetermined command or data from a user. For example, the input unit may generate and output a screen image for moving a location of the first unit from among the plurality of units.

In addition, the input unit may receive an input of a predetermined command or data from the user via the user interface screen. For example, the input unit may receive an input of a movement value of an X-axis, a movement value of a Y-axis, and a movement value of a Z-axis for moving the first unit. In more detail, via the user interface screen, a manipulation signal due to a user touch input by using various input tools may be input.

The user may visually recognize predetermined information by watching the user interface screen displayed on the display device 630, and may input the predetermined command or the data via the input unit. For example, the input unit may be formed as a touchpad. In more detail, the input unit may include a touchpad combined with a display panel included in the display device 630. In this case, the user interface screen is output onto the display panel. When a predetermined command is input via the user interface screen, the touchpad senses that and transmits sensed information to the controller 620. Afterward, the controller interprets the sensed information, and may recognize and execute the predetermined command input by the user.

The display device 630 may display a screen image for changing a setting status or an operational state of the first unit from among the plurality of units of the X-ray apparatus. In this regard, a window including a parameter for adjusting the first unit and an input column for changing a value of the parameter may be displayed on a screen. The input unit may receive a user input for controlling the setting status or the operational state of the first unit. The controller 620 may control the setting status or the operational state of the first unit, based on the user input.

In addition, the monitoring device 600 may further include a storage (not shown) and a communication unit (not shown). The storage (not shown) may store data about the plurality of units of the X-ray apparatus (e.g., parameters about the plurality of units, values of the parameters, etc.), and data transmitted from an external apparatus to the monitoring device 600. The data transmitted from the external apparatus may include initial setting values of the plurality of units of the X-ray apparatus, setting values according to usage modes of the plurality of units, or the like.

The communication unit (not shown) may receive data from the external apparatus and/or may transmit data to the external apparatus. In this case, a network used in an exchange of data with the external apparatus may be different from a network connected between the plurality of units of the X-ray apparatus. In this regard, the external apparatus may be an apparatus including all computing apparatuses, etc., which obtains, stores, processes, or uses data for controlling the X-ray apparatus.

The monitoring device 600 includes a central processing unit, thereby generally controlling operations of the port 610, the controller 620, and the display device 630. The central processing unit may be implemented as an array of a plurality of logic gates, and may be implemented as a combination of a general-use microprocessor and a memory storing a program to be executed by the microprocessor. However, it is obvious to one of ordinary skill in the art of the present embodiment that the central processing unit may be implemented as a different type of hardware.

Hereinafter, various operations or applications performed by the monitoring device 600 will now be described, and features that may be clearly understood and predicted by one of ordinary skill in the art to which this disclosure belongs may be considered as common implementations even if one of the port 610, the controller 620, and the display device 630 is not specified, and the scope of the present disclosure is not limited by a name or physical/logical structures of a particular configuration.

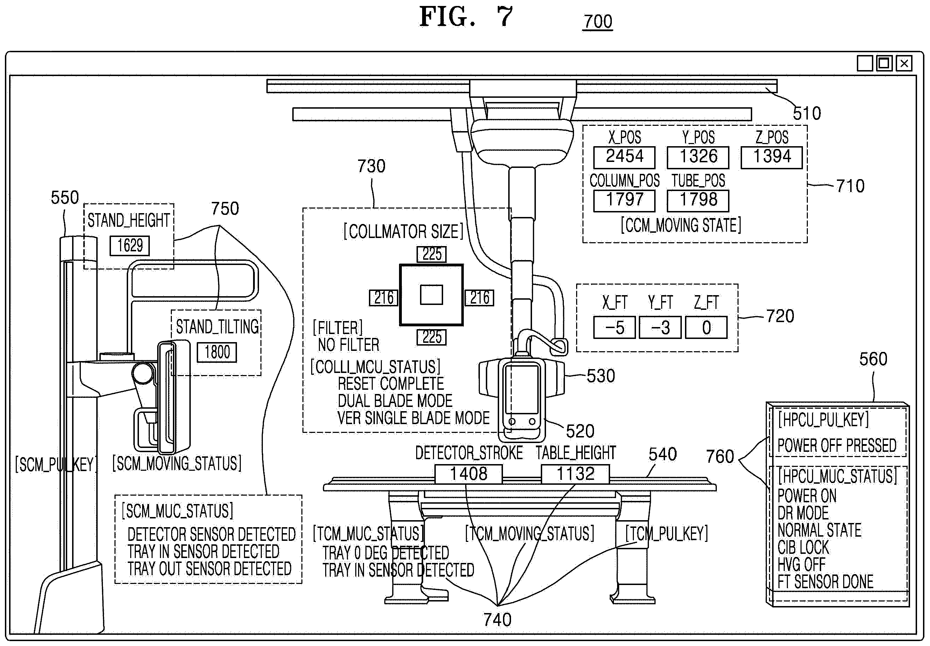

FIG. 7 is a diagram for describing a screen image displaying a monitoring result, according to an embodiment.

As illustrated in FIG. 7, the monitoring device 600 may display the monitoring result about the plurality of units. In this regard, the monitoring device 600 may display the plurality of units by displaying the plurality of units on the screen image based on actual placement of the plurality of units. In addition, the monitoring device 600 displays, on the screen image, the plurality of units in icons respectively corresponding to the plurality of units or in reduced model diagrams, so that the user may intuitively recognize an operational state of each of the plurality of units.

In addition, the monitoring device 600 may display data of each of the plurality of units displayed on the screen image in the vicinity of a corresponding unit. The monitoring device 600 may receive data of each of the plurality of units from each of the plurality of units in real-time, and may display the data.

As illustrated in FIG. 7, the X-ray apparatus may include the ceiling 510, the THU 520, the collimator 530, the table 540, the stand 550, and the HPCU 560. The X-ray apparatus may be embodied with more elements than the shown elements or may be embodied with fewer elements than the shown elements. The number of the units of the X-ray apparatus illustrated in FIG. 7, whether to display data of the units, where to display the data, or the like may be changed according to settings by the user.

Referring to FIG. 7, the monitoring device 600 may display (710) an x-coordinate value, a y-coordinate value, and a z-coordinate value of the ceiling unit 510 on the screen image, and may display (730) information indicating a size of a collimator and state information of the collimator on the screen image.

The monitoring device 600 may display (740) information indicating a location, a height, and an operational state of a table on the screen image, and may display (750) information indicating a height and a tilt of a detector on a stand, and operational states of the detector and the stand on the screen image. In addition, the monitoring device 600 may display (760) information indicating an operational state of an HPCU.

In the present embodiment, data or information about the plurality of units is illustrated as in FIG. 7, but it is obvious to one of ordinary skill in the art that the data or the information about the plurality of units may be displayed in another manner.

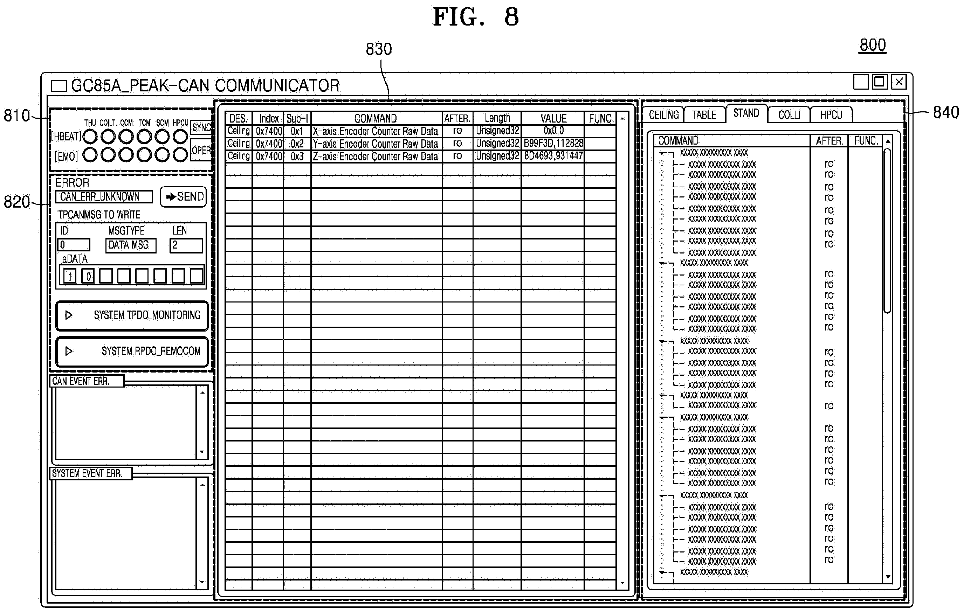

FIG. 8 is a diagram for describing a screen image displaying a monitoring result, according to another embodiment.

The monitoring device 600 may display the monitoring result about the plurality of units. The monitoring result may indicate, but is not limited to, a setting status or an operational state of at least one unit from among the plurality of units.

As illustrated in FIG. 8, the monitoring device 600 may display, on the screen image, whether each of the plurality of units normally operates. The monitoring device 600 may simply display (810), by using colors, whether each of the plurality of units normally operates. For example, if a first unit normally operates, an icon indicating a state of the first unit may be displayed using a green color. On the other hand, if the first unit does not normally operate, the icon indicating the state of the first unit may be displayed using a red color. If an inspection of the first unit is required, the icon may be displayed using a yellow color.

As illustrated in FIG. 8, when an error occurs in driving the X-ray apparatus, the monitoring device 600 may display (820) information about a unit in which the error occurred. In addition, the monitoring device 600 may differently display an error message of a case in which an error occurs in the monitoring device 600 from an error message of a case in which an error occurs in the X-ray apparatus.

The monitoring device 600 may display (830) setting statuses and operational states of units that are not displayed on the screen image. In addition, the monitoring device 600 may display setting statuses and operational states of units that are displayed on the screen image. The monitoring device 600 may display (840) a plurality of pieces of detail information about units distinguished therebetween.

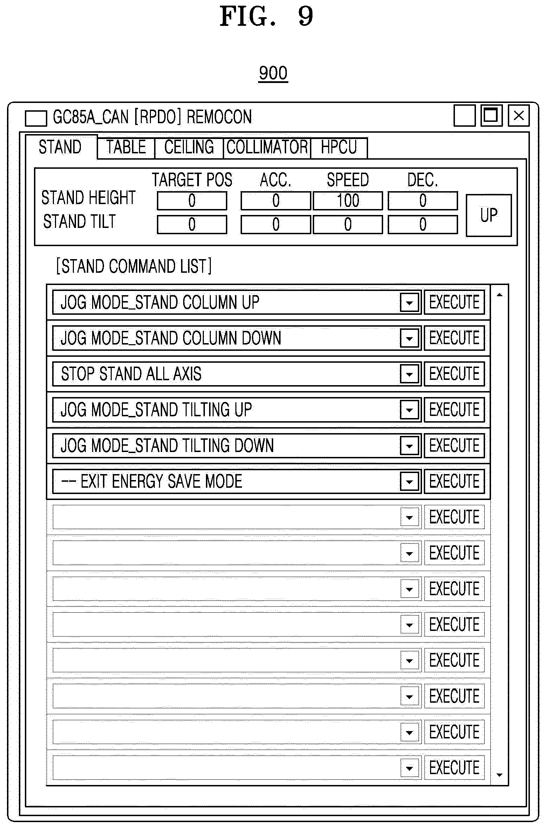

FIG. 9 is a diagram for describing a screen image for receiving a user input of controlling the X-ray apparatus, according to an embodiment.

The monitoring device 600 may receive a user input of controlling the plurality of units. The monitoring device 600 may display, on a screen, a user interface for receiving the user input of controlling the plurality of units.

As illustrated in FIG. 9, the monitoring device 600 may display, on the screen, a user interface for controlling a stand unit. A user may input information about mode setting, a location, a tilt, or the like about the stand unit, and the monitoring device 600 may control an operation of the stand unit based on the user input.

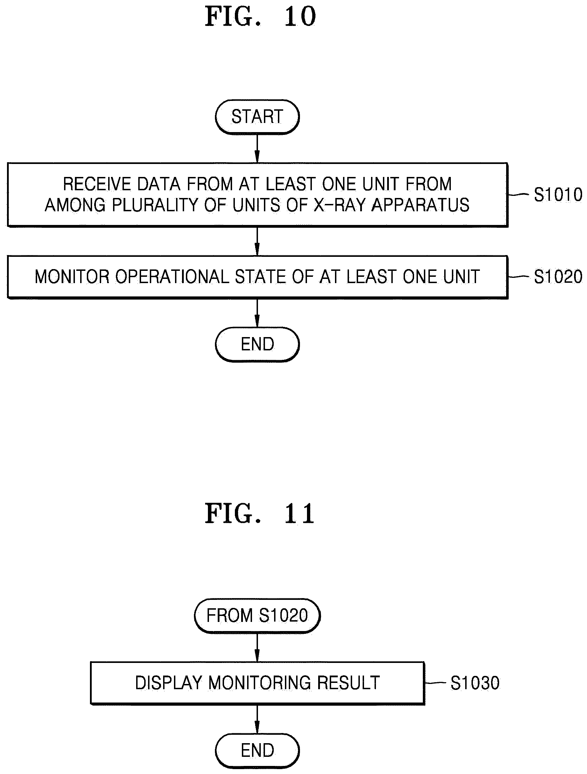

FIG. 10 is a flowchart of an operating method performed by a monitoring device, according to an embodiment.

Referring to FIG. 10, in operation S1010, the monitoring device 600 may receive data from at least one unit from among the plurality of units of the X-ray apparatus. The data may include information indicating an operational state of the at least one unit. The information indicating an operational state may include at least one of qualitative information and quantitative information. For example, the qualitative information may include information indicating whether a first unit normally operates. The quantitative information may include information indicating a parameter related to the first unit and current values of the parameter.

In operation S1020, the monitoring device 600 may monitor the operational state of the at least one unit. The monitoring device 600 may receive first data including an operational state of the first unit, and may determine the operational state of the first unit, based on the received first data. The monitoring device 600 may generate an operation command with respect to the first unit, according to the determined operational state.

For example, if a center value of the collimator of the X-ray apparatus and a center value of the detector do not match with each other, the X-ray apparatus cannot normally capture an X-ray image, so that the center value of the collimator and the center value of the detector have to match with each other. In this regard, it is assumed that the center value of the collimator and the center value of the detector do not match with each other. The monitoring device 600 receives coordinate values of the collimator or the detector. Based on the received coordinate values, the monitoring device 600 determines that an operation of the collimator or the detector does not normally operate. The monitoring device 600 may generate an operation command for moving the collimator or an operation command for moving the detector so as to make the center value of the collimator and the center value of the detector match with each other.

FIG. 11 is a flowchart of an operating method performed by the monitoring device, according to another embodiment.

Operation S1030 of FIG. 11 may be performed after S1020 of FIG. 10, and an order of operations is exemplary.

In operation S1030, the monitoring device 600 displays a monitoring result of monitoring operational states of the plurality of units. The monitoring device 600 may display a screen image displaying the plurality of units of the X-ray apparatus, and may display, on the screen image, a plurality of items of data about the plurality of units, respectively.

In this regard, the plurality of units displayed on the screen image are arranged based on actual placement of the plurality of units. That is, the plurality of units may be arranged on the screen image so as to be equal to the actual placement of the plurality of units which is viewed by a user. In addition, each of the units on the screen image may be displayed in a model diagram. Thus, the user may easily recognize the operational states of the plurality of units by the screen image displayed by the monitoring device 600.

The monitoring device 600 may display data of all parameters for the units or may display data of some of the parameters. The monitoring device 600 may display data of only a unit that does not normally operate from among the plurality of units.

In addition, the monitoring device 600 may display a screen image displaying a setting status or an operational state of at least one unit from among the plurality of units.

The monitoring device 600 may display and output various information on a screen via a graphical user interface (GUI), the various information being processed by the monitoring device 600. The monitoring device 600 may include at least two displays according to embodiments.

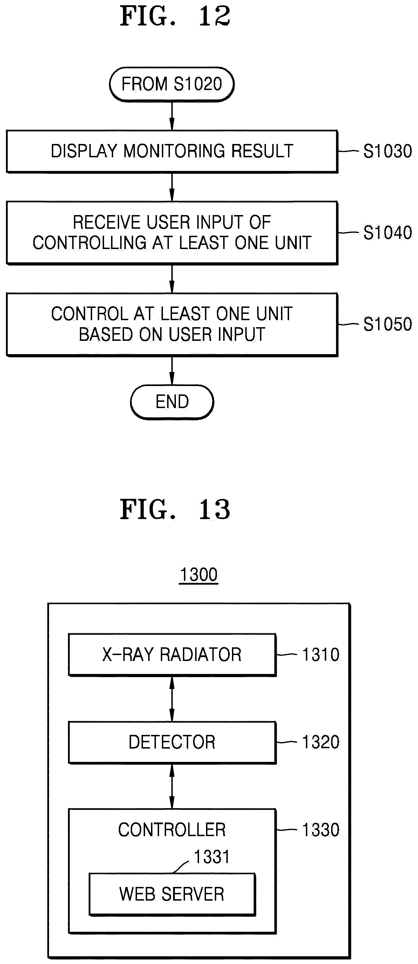

FIG. 12 is a flowchart of an operating method performed by the monitoring device, according to another embodiment.

Operation S1030 of FIG. 12 may be performed after S1020 of FIG. 10, and an order of operations is exemplary. It is obvious to one of ordinary skill in the art of the present disclosure that the monitoring device 600 can perform operations S1030 through S1050 according to another order.

In operation S1030 of FIG. 12, the monitoring device 600 displays a monitoring result of monitoring operational states of the plurality of units. Operation S1030 of FIG. 12 is equal to operation S1030 of FIG. 11, and redundant descriptions thereof are omitted here.

In operation S1040, the monitoring device 600 receives a user input of controlling at least one unit. The monitoring device 600 receives the user input of controlling a setting status or an operational state of the at least one unit. As the result of the monitoring, even if the monitoring device 600 normally operates, an operating method performed by the X-ray apparatus may vary according to users of the X-ray apparatus. In this case, the user may change a setting value or an operation value of the at least one unit so as to allow the X-ray apparatus to operate according to user intention. The setting value or the operation value of the at least one unit which is changed by the user is stored in the monitoring device 600 and may be used when the user drives the X-ray apparatus next time.

In operation S1050, the monitoring device 600 controls the at least one unit based on the user input. In more detail, as the result of the monitoring, if the X-ray apparatus does not normally operate, the monitoring device 600 may receive a user input for controlling a unit that does not normally operate. Based on the user input, the monitoring device 600 may control the unit that does not normally operate.

FIG. 13 is a block diagram illustrating a configuration of an X-ray apparatus, according to an embodiment.

According to an embodiment, an X-ray apparatus 1300 may include an X-ray radiator 1310, a detector 1320, and a controller 1330. The X-ray apparatus 1300 may be embodied with more elements than the shown elements or may be embodied with fewer elements than the shown elements. Hereinafter, the elements are sequentially described.

The X-ray radiator 1310 radiates an X-ray, and the detector 1320 detects the X-ray that has been radiated from the X-ray radiator 1310 and transmitted through an object. The controller 1330 controls the X-ray radiator 1310 to adjust a radiated X-ray, and controls the detector 1320 to detect the X-ray.

The X-ray radiator 1310 illustrated in FIG. 13 may equally correspond to the X-ray radiator 120 of the X-ray apparatus 100 illustrated in FIG. 1, and the detector 1320 illustrated in FIG. 13 may equally correspond to the detector 130 of the X-ray apparatus 100 illustrated in FIG. 1. In addition, the controller 1330 illustrated in FIG. 13 may equally correspond to the controller 150 of the X-ray apparatus 100 and the controller 113 of the workstation 110 illustrated in FIG. 1. In this regard, redundant descriptions with reference to FIG. 1 are omitted here.

A web server 1331 providing at least one of setting information, operational state information, and control information, which are respect to the X-ray apparatus 100, may be installed in the controller 1330. The web server 1331 provides a web interface for manipulating the X-ray apparatus 1300. In addition, the web server 1331 provides different web interfaces according to groups of users using the X-ray apparatus 1300. By providing the different web interfaces according to the groups of users, the X-ray apparatus 1300 may provide user-customized services.

The X-ray apparatus 1300 may further include a display (not shown) and an input unit (not shown). The display displays a web interface, and the input unit receives a user input via the web interface. The controller 1330 controls the X-ray apparatus 1300 based on the user input.

The X-ray apparatus 1300 may include a central processing unit, thereby generally controlling operations of the X-ray radiator 1310, the detector 1320, and the controller 1330. The central processing unit may be implemented as an array of a plurality of logic gates, and may be implemented as a combination of a general-use microprocessor and a memory storing a program to be executed by the microprocessor. However, it is obvious to one of ordinary skill in the art of the present embodiment that the central processing unit may be implemented as a different type of hardware.

Hereinafter, various operations or applications performed by the X-ray apparatus 1300 will now be described, and features that may be clearly understood and predicted by one of ordinary skill in the art to which this disclosure belongs may be considered as common implementations even if one of the X-ray radiator 1310, the detector 1320, and the controller 1330 is not specified, and the scope of the present disclosure is not limited by a name or physical/logical structures of a particular configuration.

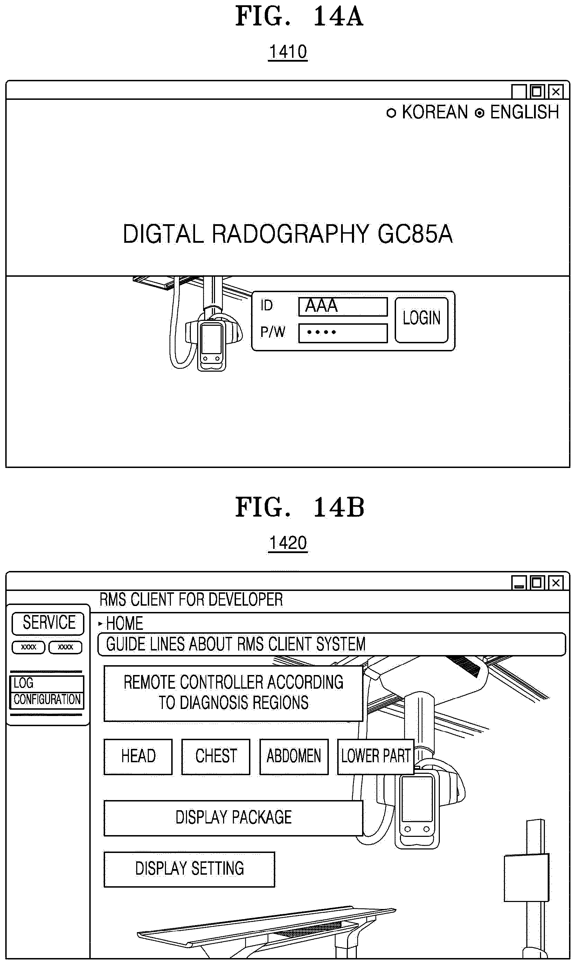

FIG. 14A is a diagram for describing a web interface provided by a web server, according to an embodiment.

As illustrated in at 1410 of FIG. 14A, before an access to the web server 1331, the web server 1331 requires user authentication. It is possible to access the web server 1331 by performing user authentication through a web browser or a workstation, a portable terminal, or an external personal computer (PC) which has an application corresponding to the web browser. In addition, the web server 1331 provides set services according to user accounts. The user accounts may be divided to groups. For example, the user accounts may be divided to a group of doctors generally using the X-ray apparatus 1300, a group of manufacturers producing the X-ray apparatus 1300, or the like, and it is obvious to one of ordinary skill in the art of the present disclosure that another group may be further included.

FIG. 14B is a diagram for describing a web interface provided by the web server, according to another embodiment.

As illustrated in at 1420 of FIG. 14B, the web server 1331 provides a web interface with respect to a user who captures an image of a patient by using the X-ray apparatus 1300. The web interface may provide information to be set to a remote controller according to diagnosis regions of the patient. For example, the diagnosis regions may include, but are not limited to, a head, a chest, an abdomen, a lower part, or the like.

When the user captures an image of a head of the patient, as illustrated in FIG. 14B, the user may select an icon of "head" in the web interface, and thus may receive a service of a method of controlling an operation of the X-ray apparatus 1300, or a method of setting the X-ray apparatus 1300.

In addition, when the user selects the icon of "head" via the input unit of the X-ray apparatus 1300, the X-ray apparatus 1300 may operate according to a pre-set process. When the user selects the icon of "head" via the input unit of the X-ray apparatus 1300, a portable terminal such as the remote controller may receive service information of the X-ray apparatus 1300, and the user may control an operation of the X-ray apparatus 1300 by manipulating the remote controller.

In addition, the web interface may provide settings for displaying a captured image of the patient. For example, it may be required to magnify an image of an important region such as a diseased area of the patient, so that the image may be magnified and displayed. When it is required to display sequential images, sequentially-captured images may be displayed. Thus, the user may previously set or change settings of the display according to the aforementioned method.

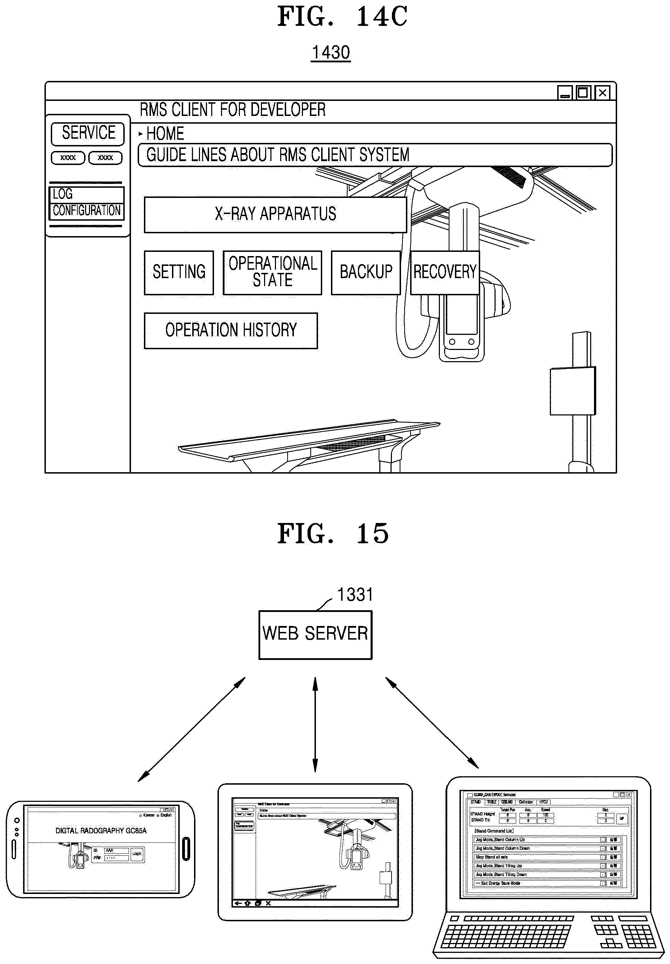

FIG. 14C is a diagram for describing a web interface provided by the web server, according to another embodiment.

As illustrated in at 1430 of FIG. 14C, the web server 1331 provides a web interface with respect to a user who changes or initializes a setting environment of the X-ray apparatus 1300. The web interface may provide a current setting value set to each unit of the X-ray apparatus 1300, and information about a current operational state of the X-ray apparatus 1300. In addition, the web interface may provide services including a backup process, a system recovery, an operation history of the X-ray apparatus 1300, or the like.

Although not illustrated in FIGS. 14B and 14C, the web server 1331 may provide a web interface for another user other than the aforementioned user. The web server 1331 may provide a web interface for a manufacturer of the X-ray apparatus 1300, thereby receiving, via the web interface, information required when the manufacturer sets production specification of the X-ray apparatus 1300.

In addition, the web server 1331 may provide a web interface for a user who repairs the X-ray apparatus 1300. When the X-ray apparatus 1300 goes wrong, a repairer for the X-ray apparatus 1300 may diagnose a failure via the web server 1331. The repairer for the X-ray apparatus 1300 may remotely perform a repair on the X-ray apparatus 1300 via the web server 1331.

FIG. 15 is a diagram for describing types of a service provided by the web server, according to an embodiment.

As illustrated in FIG. 15, an external apparatus may access the web server 1331 and may receive a service provided by the web server 1331. In this regard, the external apparatus is an apparatus to obtain, store, process or use data related to the X-ray apparatus 1300 or an X-ray image, and may correspond to a medical imaging apparatus, a medical server, a portable terminal, or all computing apparatuses capable of using and processing a medical image. For example, the external apparatus may be a medical diagnosis apparatus included in a medical institution such as a hospital. In addition, the external apparatus may be a server included in a hospital so as to record and store treatment histories of patients, a medical imaging apparatus in a hospital for a doctor to analyze a medical image, or the like.

In addition, the external apparatus may be a storage apparatus. The storage apparatus may include all of a hard disk drive (HDD), a read-only memory (ROM), a random-access memory (RAM), a flash memory, and a memory card.

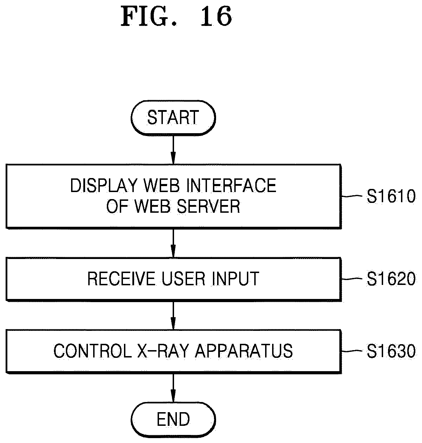

FIG. 16 is a flowchart of an operating method performed by the X-ray apparatus, according to an embodiment.

Referring to FIG. 16, in operation S1610, the X-ray apparatus 1300 displays a web interface of the web server 1331. In this regard, the web server 1331 provides, but is not limited to, at least one of setting information, operational state information, and control information which are with respect to the X-ray apparatus 1300. In addition, the web server 1331 may be installed in the controller of the X-ray apparatus 1300. The web server 1331 may provide a web interface for manipulating the X-ray apparatus 1300, and may provide different web interfaces according to groups of users.

In operation S1620, the X-ray apparatus 1300 receives a user input of manipulating the X-ray apparatus 1300 via the web interface. A user may receive a user-required service via the web interface.

In operation S1630, the X-ray apparatus 1300 controls the X-ray apparatus 1300 based on the user input.

In more detail, the user of the X-ray apparatus 1300 accesses the web server 1331 installed in the X-ray apparatus 1300. When the user accesses, a web interface based on a user account is provided. The user may select a category of a user-required service in the provided web interface, and may input a command of controlling the X-ray apparatus 1300 in the category. The X-ray apparatus 1300 receives the user-input command, and controls the X-ray apparatus 1300 according to the received command.

The above-described apparatus may be implemented by using a hardware component, a software component, and/or a combination of a hardware component and a software component. For example, the apparatus and the component described in the exemplary embodiments may be implemented by using one or more general-purpose computers or a special-purpose computers such as, for example, a processor, a controller, an arithmetic logic unit (ALU), a digital signal processor, a microcomputer, a field programmable array (FPA), a programmable logic unit (PLU), a microprocessor, or any device that may execute an instruction and respond thereto.

A processor may execute an operating system (OS) and one or more software applications executed on the OS. Also, the processor may access, store, manipulate, process, and generate data in response to execution of software.

For convenience of understanding, though description has been made to the case where one processor is used, a person of ordinary skill in the art will understand that the processor may include a plurality of processing elements and/or processing elements having a plurality of types. For example, the processor may include a plurality of processors, or one processor and one controller. Also, the processor may include a different processing configuration such as a parallel processor.

Software may include a computer program, a code, an instruction, or a combination of one or more of these, and configure the processor to operate as desired, or instruct the processor independently or collectively.

Software and/or data may be embodied permanently or temporarily in a certain type of a machine, a component, a physical device, virtual equipment, a computer storage medium or device, or a transmitted signal wave in order to allow the processor to analyze the software and/or data, or to provide an instruction or data to the processor. Software may be distributed on a computer system connected via a network, and stored and executed in a distributed fashion. Software and data may be stored in one or more non-transitory computer-readable recording media.

The methods according to exemplary embodiments may be embodied in the form of program commands executable through various computer means, which may be recorded on a non-transitory computer-readable recording medium. The non-transitory computer-readable recording medium may include program commands, data files, and data structures either alone or in combination. The program commands recorded on the non-transitory computer-readable recording medium may be those that are especially designed and configured for the inventive concept, or may be those that are known and available to computer programmers skilled in the art.

Examples of the non-transitory computer-readable recording medium include magnetic recording media such as hard disks, floppy disks, and magnetic tapes, optical recording media such as CD-ROMs and DVDs, magneto-optical recording media such as floptical disks, and hardware devices such as ROMs, RAMs, and flash memories that are especially configured to store and execute program commands.

Examples of the program commands include machine language codes that may be generated by a compiler, and high-level language codes that may be executed by a computer by using an interpreter.

The above hardware device may be configured to operate as one or more software modules in order to perform an operation of an exemplary embodiment, and vice versa.

Though the exemplary embodiments have been described by a limited number of exemplary embodiments and drawings, a person of ordinary skill in the art will make various modifications and changes from the above exemplary embodiments. For example, even when the described technologies are performed in an order different from the described method and/or components such as the described system, structure, apparatus, and circuit are coupled or combined in a form different from the described method, or replaced by other components or equivalents thereof, a proper result may be accomplished.

Therefore, the scope of the inventive concept should not be limited and determined by the described exemplary embodiments, but should be determined by not only the following claims but also equivalents thereof.

## Claims

1. A monitoring device comprising;
a port connected to a network interconnecting a plurality of units of an X-ray apparatus, and configured to transmit or receive data to or from each of the plurality of units; and
a controller configured to control the port to perform communication with each of the plurality of units, and to monitor operational states of the plurality of units, based on the data.

2. The monitoring device of claim 1, wherein the controller is further configured to control the port to receive first data comprising an operational state of a first unit from among the plurality of units.

3. The monitoring device of claim 2, wherein the controller is further configured to determine the operational state of the first unit, based on the first data, and to generate an operation command for the first unit according to the determined operational state.

4. The monitoring device of claim 3, wherein the port is further configured to transmit the operation command for the first unit to the first unit.

5. The monitoring device of claim 1, wherein the data uses at least one protocol from among a controller area network (CAN), a local interconnect network (LIN), a FlexRay, and a media oriented system transport (MOST).

6. The monitoring device of claim 1, further comprising a display device configured to display the monitoring result.

7. The monitoring device of claim 6, wherein the display device is further configured to display a screen image displaying the plurality of units of the X-ray apparatus on a screen of the monitoring device, and to display the data on the screen image.

8. The monitoring device of claim 7, wherein the plurality of units displayed on the screen image are arranged based on actual placement of the plurality of units.

9. The monitoring device of claim 6, wherein the display device is further configured to display a screen image displaying a setting status or an operational state of at least one unit from among the plurality of units.

10. The monitoring device of claim 9, wherein
the display device comprises an input unit configured to receive a user input of controlling the setting status or the operational state, and
the controller is further configured to control the setting status or the operational state of the at least one unit, based on the user input.

11. The monitoring device of claim 1, wherein the port is connected to a link to a first unit from among the plurality of units.

12. The monitoring device of claim 1, further comprising a communication unit configured to transmit the data to an external apparatus via another network different from the network.

13. An X-ray apparatus comprising:
an X-ray radiator configured to radiate an X-ray;
a detector configured to detect the X-ray that has been radiated from the X-ray radiator and transmitted through an object; and
a controller configured to control the X-ray radiator to adjust the radiated X-ray, and to control the detector to detect the X-ray,
wherein a web server configured to provide at least one of setting information, operational state information, and control information, which are with respect to the X-ray apparatus, is installed in the controller.

14. The X-ray apparatus of claim 13, wherein the web server is further configured to provide different web interfaces according to groups of users using the X-ray apparatus.

15. The X-ray apparatus of claim 14, further comprising:
a display configured to display the web interfaces; and
an input unit configured to receive a user input via the web interfaces,
wherein the controller is further configured to control the X-ray apparatus, based on the user input.
